# FASCICULE DE BREVET EUROPEEN

(11) **EP 3 075 866 B1**
(45) Date de publication et mention de la délivrance du brevet: **24.04.2019**
(21) Numéro de dépôt: 16163476.1
(22) Date de dépôt: 01.04.2016
(51) Int. Cl.: C12Q 1/68, G01N 1/28, G01N 35/00, C12N 15/10, B01L 7/00

(54) **INSTALLATION POUR LE TRAITEMENT AUTOMATISE DES DONNÉES D'UN ROBOT D' EXTRACTION DES ACIDES NUCLÉIQUES ET D'AMPLIFICATION DE GÈNES PAR LE PROCÉDÉ PCR TEMPS RÉEL**
ANLAGE ZUR AUTOMATISIERTEN DATENVERARBEITUNG EINES ROBOTERS ZUR EXTRAKTION VON NUKLEINSÄUREN UND ZUR GENAMPLIFIKATION MITTELS ECHTZEIT-PCR-VERFAHREN
SYSTEM FOR AUTOMATED PROCESSING OF DATA FROM A ROBOT FOR EXTRACTING NUCLEIC ACIDS AND AMPLIFYING GENES BY THE REAL-TIME PCR METHOD

(30) Priorité: 01.04.2015 FR 1552812
(43) Date de publication de la demande: 05.10.2016
(73) Titulaire: AD NUCLEIS, 69290 Grezieu-la-Varenne (FR)
(72) Inventeur: FRANCK, Michel, 69290 Grezieu-la-Varenne (FR); FRANCK, Pascal, 69126 Brindas (FR); LEQUETTE, Yannick, 69340 Francheville (FR); LAWSON, Nadou, 69009 Lyon (FR); FRANCK, Catherine, 69290 Grezieu-la-Varenne (FR)
(74) Mandataire: Verriest, Philippe

(56) Documents cités:
- EP-A1- 2 628 786
- WO-A2-2005/014850
- US-A1- 2011 294 205
- US-A1- 2014 186 236

## Description

### Domaine de l'invention

Le domaine de la présente invention concerne :
a) celui de la détection et éventuellement de la quantification de l'acide désoxyribonucléique (par la suite appelée ADN) et/ou de l'acide ribonucléique (par la suite appelé ARN et transformé par une enzyme en ADN) de tout organisme vivant eucaryote ou procaryote, appelé « cible », dans les produits biologiques tels que le sang, l'urine, les produits laitiers, les produits carnés, les produits liquides, les eaux claires, les eaux usées, les céréales et tous les végétaux, les aliments pour animaux; ces cibles peuvent être pathogènes ou non pathogènes ;
b) et plus notamment un procédé de détection et/ou de quantification entièrement automatisé de la ou des cibles ;
c) l'ensemble étant intégré dans un appareil du type « boîte noire » (connue sous le nom de « black box » en anglais) permettant l'analyse industrielle des échantillons en vue du contrôle de leur état sanitaire, y compris les allergènes, les organismes génétiquement modifiés (par la suite appelée OGM), les résistances aux antibiotiques, ou de leur appartenance à une espèce, ou de leur composition en ADN microsatellites aux fins de détermination de la filiation ou de la parenté de l'animal ou du végétal dont il est issu.

### Art antérieur

De nos jours, la présence de cibles dans les produits consommables, tels que les boissons, les produits alimentaires, les réseaux d'eau potable ou d'eau industrielle, engendre de réels risques pour la santé des êtres vivants et en particulier pour la santé des êtres humains. Les produits alimentaires contiennent ainsi, pour certains, des cibles tels que des virus, des bactéries (Salmonelles, Listeria monocytogenes, etc...), des champignons, des levures, des parasites, des allergènes, qui sont susceptibles de provoquer diverses maladies chez l'être humain ou de tromper le consommateur dans les cas de fraude alimentaire. Parmi ces maladies, on peut citer entre autres, la légionellose ou encore la (les) gastro-entérite(s) virale(s) ou bactérienne(s).

Étant donné la présence de diverses cibles potentielles dans un produit alimentaire, ce dernier doit être contrôlé sur son authenticité et ses qualités sanitaires et éventuellement décontaminé, avant de pouvoir être considéré comme répondant aux normes des organismes de sécurité alimentaire et ce dans un temps relativement court afin de ne pas gêner une production et/ou la récolte de produits alimentaires.

Plus généralement, les cibles recherchées peuvent être ou non pathogènes ; les cibles non pathogènes appelées parfois « indicateurs d'hygiène » sont prioritairement visées, en ce sens qu'elles caractérisent l'état d'hygiène de l'échantillon ; sont recherchées aussi la présence ou non d'allergènes, d'OGM, de résistances aux antibiotiques ou l'appartenance à telle ou telle espèce.

Il existe donc un besoin de mettre au point un procédé industriel ainsi qu'un dispositif de détection et/ou de dosage permettant de révéler la présence qualitative et/ou quantitative de cibles à moindre coût et avec une sensibilité élevée avec un résultat le plus proche des méthodes ISO et en particulier des méthodes bactériologiques de référence ISO ; le procédé industriel de l'opération constitue le coeur de l'innovation en raison du nombre important de tests à réaliser dès que les indicateurs d'hygiène sont recherchés; à ce jour la carence de procédés visant la recherche et la quantification des indicateurs d'hygiène par la méthode PCR est grande ; le présent brevet est une réponse à cette carence.

### Exposé de l'invention

La présente divulgation a pour but de résoudre tout ou partie des inconvénients mentionnés ci-dessus sous la forme d'un dispositif industriel destiné à traiter par un automate les échantillons contenant de l'acide nucléique en suivant un processus d'extraction, de purification par séparation, d'amplification pour la détection et/ou le dosage d'acide nucléique de cible(s) dans au moins un échantillon d'au moins une matrice, ledit au moins un échantillon étant contenu dans un organe de réception destiné à être placé dans une enceinte ; ladite enceinte comprend des parois latérales, un plafond, un plancher, une ouverture ; ladite enceinte est configurée pour automatiser la manipulation de l'échantillon à l'intérieur de l'enceinte du dispositif d'extraction de purification par séparation, d'amplification pour la détection et/ou de dosage du ou des acides nucléiques; ledit dispositif d'extraction, de purification par séparation, d'amplification pour la détection et/ou de dosage comprend :
- au moins un organe de transfert ; l'organe de transfert est configuré pour transférer au moins une partie de l'échantillon dans l'enceinte ;
- au moins un dispositif d'entraînement; le dispositif d'entraînement est configuré pour permettre le déplacement de l'organe de transfert et/ou de l'organe de réception dans l'enceinte ;
- au moins un séparateur; le séparateur est configuré pour séparer les cibles à détecter et/ou à doser dans l'échantillon ;
- au moins un homogénéisateur; l'homogénéisateur est configuré pour homogénéiser les cibles à détecter et/ou à doser dans un liquide d'homogénéisation ;
- au moins une scelleuse ; la scelleuse est configurée pour sceller un organe de réception ; et
- au moins un thermocycleur temps réel ; le thermocycleur temps réel est configuré pour permettre une réaction en chaîne par polymérase.

Selon un mode de réalisation, le thermocycleur temps réel comprend en outre une chambre.

Selon un mode de réalisation, le dispositif de transfert est configuré pour permettre la migration de l'organe de réception au sein de l'enceinte ou vers la chambre du thermocycleur temps réel.

Grâce à cette disposition, le dispositif de transfert permet de transférer l'organe de réception de l'enceinte vers la chambre du thermocycleur temps réel sans que l'utilisateur ait besoin de manipuler l'organe de réception.

Selon un mode de réalisation, le thermocycleur temps réel comprend :
- un ensemble de dispositifs de chauffage et de refroidissement; l'ensemble de dispositifs de chauffage et de refroidissement permet de faire varier la température de l'organe de réception entre plusieurs cycles de températures ;
- un ensemble de blocs mobiles de chauffage ; l'ensemble de blocs mobiles de chauffage comprend une surface de couplage configurée pour permettre le chauffage de l'organe de réception ;
- un dispositif de mesure ; le dispositif de mesure comprend une source de lumière et un détecteur configuré pour permettre la détection de la lumière émise par les substances chimiques appelées fluorophores et qui ont pour propriété d'émettre une fluorescence de longueur d'onde précise lorsqu'ils reçoivent une excitation par une source de lumière dite d'excitation et dont la longueur d'onde est spécifique pour chaque fluorophore.

Grâce à cette disposition, le thermocycleur peut mesurer en temps réel une quantité de lumière et par la même occasion les gammes de longueurs d'ondes émises si plusieurs fluorophores sont utilisés.

Selon un mode de réalisation, la source de lumière est configurée pour permettre la fluorescence de substances fluorophores.

Selon un mode de réalisation, la source de lumière est configurée pour permettre l'excitation spécifique de substances fluorophores.

Selon un mode de réalisation, le détecteur du dispositif de mesure est configuré pour permettre la détection d'une gamme de longueur d'onde émise par des substances fluorophores.

Selon un mode de réalisation, le détecteur du dispositif de mesure est configuré pour permettre la détection de différentes plages d'une longueur d'onde émise respectivement par les différentes substances fluorophores.

Selon un mode de réalisation, les substances fluorophores sont configurées pour émettre une fluorescence en présence de la cible et émettrices d'une fluorescence sous l'effet d'une longueur d'onde.

Selon un mode de réalisation, les substances fluorophores sont configurées pour émettre une fluorescence en présence de la cible et émettrices d'une fluorescence sous l'exposition d'une source de lumière.

Selon un mode de réalisation, la source de lumière est configurée pour émettre une lumière d'excitation selon un rayon optique.

Grâce à cette disposition, la source de lumière est focalisée selon un trajet précis.

Selon un mode de réalisation, la longueur d'onde de l'émission de la source de lumière se situe dans les UV et spectre visible, en particulier entre 300 nm et 600 nm.

Selon un mode de réalisation, la gamme de longueur d'onde de lumière absorbée par l'organe de réception est située entre les Ultra-Violet et le spectre visible, en particulier entre 400 nm et 700 nm.

Selon un mode de réalisation, le thermocycleur temps réel comprend en outre un dispositif de déviation de trajet optique.

Ainsi, grâce à cette disposition, le dispositif d'extraction du ou des acides nucléiques des cibles, de leur purification par séparation et/ou purification et/ou de dosage peut détecter et/ou doser le(s) acide(s) nucléique(s) dans un échantillon quelconque.

Selon un mode de réalisation, l'organe de réception peut contenir un milieu réactionnel.

Selon un mode de réalisation, le milieu réactionnel comprend un mix d'extraction.

Ainsi, grâce à cette disposition, l'extraction du ou des acides nucléiques des cibles en est facilité.

Selon un mode de réalisation, le milieu réactionnel comprend un mix d'amplification.

Ainsi, grâce à cette disposition, l'amplification du ou des acides nucléiques extraites des cibles en est facilité.

Selon un mode de réalisation, le mix d'amplification est configuré pour amplifier tout ou partie de l'acide nucléique extrait des cibles.

Selon un mode de réalisation, le mix d'amplification est configuré pour émettre une lumière fluorescente lorsque la cible est présente et amplifiée et lorsque le mix d'amplification est excité par la lumière d'excitation.

Ainsi, grâce à cette disposition, la présence qualitative et/ou quantitative de l'acide nucléique des cibles dans l'organe de réception est révélée par la quantification de la lumière fluorescente.

Selon un mode de réalisation, l'organe de réception comprend une plaque.

Selon un mode de réalisation, la plaque comprend un multiple de 8 de puits d'analyses.

Selon un mode de réalisation, l'organe de réception est configuré pour faciliter la transmission de chaleur entre l'intérieur de l'organe de réception et l'extérieur de l'organe de réception.

Ainsi grâce à cette disposition, l'organe de réception permet à son contenu d'être chauffé et/ou refroidi.

Selon un mode de réalisation, l'organe de réception est configuré pour faciliter la transmission d'une quantité de lumière d'excitation.

Ainsi grâce à cette disposition, l'organe de réception permet à son contenu d'être excité par une lumière d'excitation.

Selon un mode de réalisation, le contenu de l'organe de réception est configuré pour absorber une gamme de longueur d'onde de la lumière d'excitation.

Ainsi grâce à cette disposition, le contenu de l'organe de réception peut émettre une gamme de longueur d'onde autre que celle de la lumière émissive.

Selon un mode de réalisation, l'organe de transfert comprend un pipeteur.

Selon un mode de réalisation, le pipeteur comprend un consommable.

Selon un mode de réalisation, le consommable peut comprendre un cône.

Selon un mode de réalisation, le consommable peut comprendre une aiguille.

Selon un mode de réalisation, l'organe de transfert comprend un automate.

Ainsi grâce à cette disposition, l'organe de transfert peut transférer le contenu de l'organe de réception vers un autre organe de réception par exemple.

Selon un mode de réalisation, l'organe de transfert comprend un automate pipeteur.

Ainsi grâce à cette disposition, l'organe de transfert peut transférer le contenu de l'organe de réception vers un autre organe de réception par exemple de façon automatisée.

Selon un mode de réalisation, l'automate pipeteur comprend au moins un canal ; ledit canal comprend une ouverture, une cavité et un orifice d'admission configuré pour permettre l'admission d'une matière à l'intérieur de la cavité.

Ainsi grâce à cette disposition, l'organe de transfert peut transférer le contenu de l'organe de réception vers un autre organe de réception par exemple.

Selon un mode de réalisation, le canal est configuré pour permettre l'aspiration d'une matière et/ou le refoulement de la matière comprise à l'intérieur de la cavité.

Ainsi grâce à cette disposition, le canal permet de contenir un contenu pouvant transférer le contenu de l'organe de réception vers un autre organe de réception par exemple.

Selon un mode de réalisation, le séparateur comprend une centrifugeuse.

Selon un mode de réalisation, le séparateur comprend un appareil de filtration.

Selon un mode de réalisation, le séparateur comprend un appareil de purification.

Selon un mode de réalisation, le séparateur comprend une plaque aimantée.

Selon un mode de réalisation, la plaque aimantée comprend des aimants.

Selon un mode de réalisation, les aimants sont perpendiculaires à la plaque aimantée.

Ainsi grâce à cette disposition, l'organe de réception peut être disposé de façon verticale entre les aimants verticaux de la plaque aimantée.

Selon un mode de réalisation, le séparateur comprend des billes constituées par un matériau ferromagnétique et recouvertes de silice.

Ainsi grâce à cette disposition, l'ADN peut se fixer sur la silice des billes ferromagnétiques.

Selon un mode de réalisation, le séparateur comprend des billes constituées par un matériau ferromagnétique et recouverte d'un composé permettant la fixation des acides nucléiques selon des conditions physico-chimiques spécifiques.

Ainsi grâce à cette disposition, l'ADN peut se fixer sur des billes ferromagnétiques enrobées d'une substance permettant la fixation des acides nucléiques selon des conditions physico-chimiques spécifiques.

Selon un mode de réalisation, les aimants sont configurés pour fournir un champ magnétique permettant de séparer, notamment par attraction, les brins d'acides nucléiques de la ou des cibles présentes dans l'échantillon et fixés sur au moins une bille ferromagnétique.

Ainsi grâce à cette disposition, les brins d'acides nucléiques peuvent être séparés au sein du milieu réactionnel du fait de l'attraction par les aimants des billes ferromagnétiques recouvertes d'une substance permettant la fixation des acides nucléiques selon des conditions physico-chimiques spécifiques.

Selon un mode de réalisation, le champ magnétique est configuré pour permettre de séparer les brins d'acides nucléiques fixés sur la silice du reste de la solution.

Selon un mode de réalisation, le champ magnétique est configuré pour permettre de séparer les brins d'acides nucléiques fixés sur les billes ferromagnétiques.

Ainsi grâce à cette disposition, les brins d'acides nucléiques peuvent être « purifiés » dans le liquide d'homogénéisation par attraction par les aimants des billes en matériau ferromagnétique recouvertes d'une substance permettant la fixation des acides nucléiques selon des conditions physico-chimiques spécifiques.

Selon un mode de réalisation, le champ magnétique est configuré pour attirer les billes recouvertes de silice sur lesquelles sont fixés les brins d'acides nucléiques de la ou des cibles à détecter et/ou à doser.

Selon un mode de réalisation, le champ magnétique est configuré pour attirer les billes fixant les brins d'acides nucléiques de la ou des cibles à détecter et/ou à doser.

Ainsi grâce à cette disposition, les brins d'acides nucléiques peuvent être séparés en attirant les billes fixant les acides nucléiques vers et/ou dans un endroit particulier afin de les séparer d'autres composants.

Selon un mode de réalisation, le champ magnétique est configuré pour attirer les billes recouvertes de silice sur lesquelles sont fixés les brins d'acides nucléiques de la ou des cibles à détecter et/ou à doser dans le milieu réactionnel.

Selon un mode de réalisation, le champ magnétique est configuré pour attirer les billes fixant les brins d'acides nucléiques de cibles à détecter et/ou à doser dans le milieu réactionnel.

Ainsi grâce à cette disposition, les brins d'acides nucléiques peuvent être « purifiés » en les attirant afin de les séparer du milieu réactionnel.

Selon un mode de réalisation, le champ magnétique est configuré pour attirer une bille.

Selon un mode de réalisation, la bille présente une forme sphérique ou ovale.

Ainsi grâce à cette disposition, la forme sphérique de la bille permet d'une part à la bille un meilleur déplacement dans le milieu réactionnel, d'autre part une forme sans arête permettant d'accrocher plus facilement les brins d'acides nucléiques, et enfin une surface maxima de coaptation pour un volume donné permettant d'optimiser la purification de l'ADN.

Selon un mode de réalisation, la bille est une microbille.

Selon un mode de réalisation, le diamètre de la microbille est compris entre 1nm et 5000 nm, en particulier entre 2 nm et 3500 nm et notamment entre 3nm et 2000 nm.

Ainsi grâce à cette disposition, la taille de la microbille permet de faciliter l'accroche des brins d'acides nucléiques.

Selon un mode de réalisation, la bille comprend un noyau en oxyde de fer.

Ainsi grâce à cette disposition, la bille peut être attirée par un aimant.

Selon un mode de réalisation, la bille comprend une enveloppe en matériau polymère.

Ainsi grâce à cette disposition, un brin d'acide nucléique peut être accroché sur la bille.

Selon un mode de réalisation, la microbille est configurée pour être aimantée.

Ainsi grâce à cette disposition, la microbille peut être attirée par un aimant.

Selon un mode de réalisation, la microbille est configurée pour être aimantée par le champ magnétique fourni par la plaque aimantée.

Ainsi grâce à cette disposition, la microbille peut être attirée par un aimant.

Selon un mode de réalisation, la microbille est configurée pour permettre la fixation des brins d'acides nucléiques de cibles à détecter et/ou à doser.

Ainsi grâce à cette disposition, la microbille permet de filtrer des brins d'acides nucléiques d'une solution.

Selon un mode de réalisation, la microbille est configurée pour permettre la fixation des brins d'acides nucléiques de cibles à détecter et/ou à doser dans le milieu réactionnel.

Ainsi grâce à cette disposition, la microbille permet de purifier des brins d'acide nucléique d'une solution.

Selon un mode de réalisation, l'homogénéisateur comprend:
- un plateau destiné à recevoir au moins un organe de réception servant à réceptionner une quantité de d'échantillon; et
- un moteur rotatif configuré pour mettre le plateau en rotation autour d'un axe central.

Ainsi grâce à cette disposition, l'homogénéisateur permet d'homogénéiser une solution et voire même de séparer différents composants dans ladite solution.

Selon un mode de réalisation, le moteur rotatif peut mettre le plateau en rotation autour d'un centre de gravité selon un axe central.

Ainsi grâce à cette disposition, l'homogénéisateur est parfaitement centré.

Selon un mode de réalisation, le moteur rotatif est configuré pour permettre une rotation comprise entre 0 g et 9000 g.

Ainsi grâce à cette disposition, l'homogénéisateur permet de séparer différents composants d'une solution par centrifugation.

Selon un mode de réalisation, l'homogénéisateur comprend une résistance chauffante.

Ainsi grâce à cette disposition, la résistance chauffante permet de chauffer l'homogénéisateur.

Selon un mode de réalisation, l'homogénéisateur comprend un thermostat.

Grâce à cette disposition, le thermostat permet mesurer la température de l'homogénéisateur.

Selon un mode de réalisation, le thermostat est configuré pour permettre à l'homogénéisateur de se maintenir à une température.

Selon un mode de réalisation, le thermostat est configuré pour permettre à l'homogénéisateur de se maintenir à une température comprise entre 253 k et 473 k c'est-à-dire entre -20°C et +200°C.

Selon un mode de réalisation, l'enceinte comprend une station de décontamination de l'organe de transfert

Grâce à cette disposition, la station de décontamination permet de décontaminer un objet.

Selon un mode de réalisation, la station de décontamination est configurée pour éviter une contamination.

Selon un mode de réalisation, la station de décontamination est configurée pour permettre au pipeteur de changer de consommable.

Grâce à cette disposition, la station de décontamination permet au pipeteur de ne pas être contaminé.

Selon un mode de réalisation, la station de décontamination est configurée pour permettre au pipeteur de changer de cônes.

Grâce à cette disposition, la station de décontamination permet au pipeteur de ne pas être contaminé.

Selon un mode de réalisation, la station de décontamination est configurée pour permettre de décontaminer les aiguilles du pipeteur.

Grâce à cette disposition, la station de décontamination permet au pipeteur de ne pas être contaminé.

Selon un mode de réalisation, le dispositif d'extraction du ou des acides nucléiques, de leur purification par séparation et/ou de dosage des cibles comprend en outre un dispositif de transfert.

Grâce à cette disposition, le dispositif de transfert permet de transférer un objet dans l'enceinte.

Selon un mode de réalisation, le thermocycleur temps réel comprend en outre un organe d'asservissement.

Selon un mode de réalisation, le détecteur est configuré pour transmettre un signal à l'organe d'asservissement.

Ainsi grâce à cette disposition, le détecteur peut transmettre un signal à l'organe d'asservissement.

Selon un mode de réalisation, le signal du détecteur comprend une valeur de tension et/ou de courant.

Ainsi grâce à cette disposition, le détecteur peut transmettre un signal à l'organe d'asservissement.

Selon un mode de réalisation, le signal du détecteur comprend une valeur de tension et/ou de courant variable au cours du temps.

Ainsi grâce à cette disposition, le signal peut être envoyé à l'organe d'asservissement.

Selon un mode de réalisation, le thermocycleur temps réel comprend en outre un élément de chauffage.

Selon un mode de réalisation, l'élément de chauffage est configuré pour être asservi en courant par l'organe d'asservissement.

Ainsi grâce à cette disposition, l'organe d'asservissement peut contrôler la chaleur dissipée par l'élément de chauffage.

Selon un mode de réalisation, l'élément de chauffage comprend une résistance chauffante.

Selon un mode de réalisation, le thermocycleur temps réel comprend en outre un élément de refroidissement.

Selon un mode de réalisation, l'élément de refroidissement est configuré pour être asservi en tension et/ou en courant par l'organe d'asservissement.

Ainsi grâce à cette disposition, l'organe d'asservissement peut contrôler le refroidissement de son enceinte.

Selon un mode de réalisation, l'élément de refroidissement comprend un ventilateur.

Selon un mode de réalisation, le thermocycleur temps réel comprend en outre une sonde de température.

Ainsi grâce à cette disposition, le thermocycleur temps réel peut contrôler la température de son enceinte.

Selon un mode de réalisation, la sonde de température est configurée pour émettre un signal vers l'organe d'asservissement.

Selon un mode de réalisation, la sonde de température est configurée pour permettre de régler la température.

Ainsi grâce à cette disposition, le thermocycleur temps réel peut contrôler la température de son enceinte.

Selon un mode de réalisation, l'organe d'asservissement est configuré pour asservir l'élément de chauffage et/ou l'élément de refroidissement de sorte à obtenir une température de sonde de température correspondante à une valeur de consigne.

Selon un mode de réalisation, le thermocycleur temps réel réalise des cycles de températures, chaque cycle comprenant au moins deux valeurs de consigne

Selon un mode de réalisation, l'ensemble des cycles réalisé par le thermocycleur temps réel constitue un cycle thermique maîtrisé par l'organe d'asservissement

Selon un mode de réalisation, l'amplification des brins d'acide nucléique se déroule durant le cycle thermique ou cycle d'amplification

La présente divulgation concerne en outre un procédé standardisé d'extraction du ou des acides nucléiques, de leur purification par séparation, d'amplification pour la détection et/ou de dosage de cibles dans un échantillon comprenant la succession d'étapes séquentielles, lesdites étapes comprennent :
- insertion de l'échantillon dans un organe de réception ;
- ajout d'un milieu réactionnel dans l'organe de réception ;
- lyse des cibles ;
- ajout à la lyse des cellules des cibles au moins un agent chaotropique et/ou au moins un agent modificateur d'adsorption ;
- homogénéisation de la lyse par rotation et/ou par chauffage ;
- purification de l'acide nucléique de la cible ;
- concentration de brins d'acides nucléiques de cibles présents dans l'échantillon ;
- amplification d'au moins un premier brin d'acide nucléique de la cible ;
- amplification d'au moins un second brin d'acide nucléique de la cible ; et
- détection et/ou de dosage d'acide nucléique des cibles présents dans l'échantillon.

Selon un mode de réalisation, la lyse des cibles est réalisée par broyage.

Selon un mode de réalisation, la lyse des cibles est réalisée par action physique, chimique, thermique et/ou enzymatique .

Selon un mode de réalisation, les cibles détectées sont des bactéries, des virus des levures, des champignons, des parasites et tout agent pouvant contenir de l'acide nucléique.

Selon un mode de réalisation, l'agent chaotropique comprend un sel de guanidine.

Selon un mode de réalisation, l'agent chaotropique comprend du thiocyanate de guanidine.

Selon un mode de réalisation, l'organisme modificateur d'adsorption comprend un alcool.

Selon un mode de réalisation, l'organisme modificateur d'adsorption comprend de l'éthanol.

Selon un mode de réalisation, l'amplification d'acides nucléiques est réalisée par une réaction en chaine par polymérase.

Selon un mode de réalisation, le thermocycleur temps réel est configuré pour permettre la détection de tout ou partie de l'acide nucléique contenu par la cible.

Selon un mode de réalisation, la détection de tout ou partie de l'acide nucléique contenu par la cible est une détection réalisée par fluorescence, par luminescence et/ou par spectrométrie de masse ; la fluorescence est le mode de détection le plus usité

Selon un mode de réalisation, la détection de tout ou partie de l'acide nucléique contenu par la cible comprend les étapes suivantes :
- placement d'un ensemble de coordonnées uniques dans un repère orthonormé de sorte à former une courbe de détection ; la coordonnée unique comprend une intensité de signal de fluorescence normée en fonction d'une valeur unique de cycle d'amplification ; la courbe de détection dans le repère orthonormé comprend en ordonnée l'intensité de signal de fluorescence normée et en abscisse la valeur de cycle d'amplification ; et en outre ladite courbe de détection comprend une partie basse, une partie haute supérieure, en termes de valeurs d'intensité de signal de fluorescence, à la partie basse et une partie transitoire configurée pour assurer la transition entre la partie basse et la partie haute et étant supérieure, en terme de valeurs d'intensité de signal de fluorescence normées, à la partie basse et aussi inférieure, en terme de valeurs d'intensité de signal de fluorescence normée, à la partie haute ;
- constitution d'un palier bas de fluorescence à partir de la partie basse de la courbe de détection ; le palier bas de fluorescence comprend une ordonnée en intensité de fluorescence et au moins une abscisse de cycle d'amplification ;
- constitution d'un palier haut à partir de la partie haute de la courbe de détection; le palier haut de fluorescence comprend une ordonnée en intensité de fluorescence et au moins une abscisse de cycle d'amplification ;
- calcul de la différence d'intensité de fluorescence entre le palier bas et le palier haut ;
- fractionnement de la différence de fluorescence en portions proportionnelles; lesdites portions proportionnelles comprennent une ordonnée initiale et une ordonnée finale comprises entre le palier bas et le palier haut de la courbe de détection ;
- correspondance entre l'ordonnée initiale et/ou finale d'au moins une portion proportionnelle et l'intensité de signal de fluorescence de la partie transitoire la plus proche de l'ordonnée initiale et/ou finale de la portion proportionnelle ;
- sélection de la coordonnée unique correspondant à l'ordonnée initiale et/ou finale d'au moins une portion proportionnelle ;
- établissement d'une droite à partir des coordonnées uniques sélectionnées ;
- prolongement de la droite établie de sorte à déterminer un point d'intersection entre ladite droite et le palier bas et/ou le palier haut ;
- fourniture d'une valeur d'abscisse correspondant au point d'intersection.

Selon un mode de réalisation, la détection de tout ou partie de l'acide nucléique contenu par la cible comprend en outre un lissage de la courbe de détection en effectuant une moyenne de fluorescence d'un cycle d'amplification entre un cycle d'amplification le précédant et un cycle d'amplification le suivant.

Selon un mode de réalisation, la détection de tout ou partie de l'acide nucléique contenu par la cible comprend en outre une étape d'établissement d'une courbe d'étalonnage linéaire ; ladite courbe d'étalonnage comprend un repère orthonormé, un axe d'abscisse représentant la concentration de la cible et en ordonnée la valeur de l'axe des abscisses de l'intersection entre le palier bas et la droite sécante.

Selon un mode de réalisation, la courbe d'étalonnage linéaire comprend au moins une concentration connue de cibles et/ou une concentration de cibles à doser.

Selon un mode de réalisation, le procédé comprend en outre une mesure de la linéarité de la droite établie.

La présente divulgation concerne en outre un produit programme d'ordinateur comprenant des instructions de code pour l'exécution des étapes du procédé entre un dispositif selon l'invention et un terminal connecté audit dispositif.

D'autres caractéristiques et avantages de l'invention apparaîtront mieux à la lecture de la description qui suit d'un mode de réalisation de l'invention donné à titre d'exemple non limitatif.

### Liste des figures

L'invention sera mieux comprise à l'aide de la description détaillée qui est exposée ci-dessous en regard du dessin, dans lesquels :
- la figure 1 représente un exemple dans lequel le dispositif de détection et/ou de dosage selon l'invention est mis en oeuvre ;
- la figure 2 montre un exemple de courbe de détection lorsque le procédé de détection et/ou de dosage est mis en oeuvre ;
- la figure 3 illustre un exemple de constitution de paliers selon un mode de réalisation ;
- la figure 4 expose un exemple de calcul de différence entre palier selon un mode de réalisation ;
- la figure 5 présente un exemple de fractionnement de différence ;
- la figure 6 représente un exemple de correspondance entre portion proportionnelle et une partie transitoire ;
- la figure 7 montre un exemple d'établissement d'une droite ;
- la figure 8 illustre un exemple de constitution de paliers selon un mode de réalisation ; et
- la figure 9 et 10 expose des exemples de courbes d'étalonnage selon des modes de réalisations.

Dans la description détaillée qui va suivre des figures définies ci-dessus, les mêmes éléments ou les éléments remplissant des fonctions identiques pourront conserver les mêmes références de manière à simplifier la compréhension de l'invention.

### Description d'un mode de réalisation de l'invention

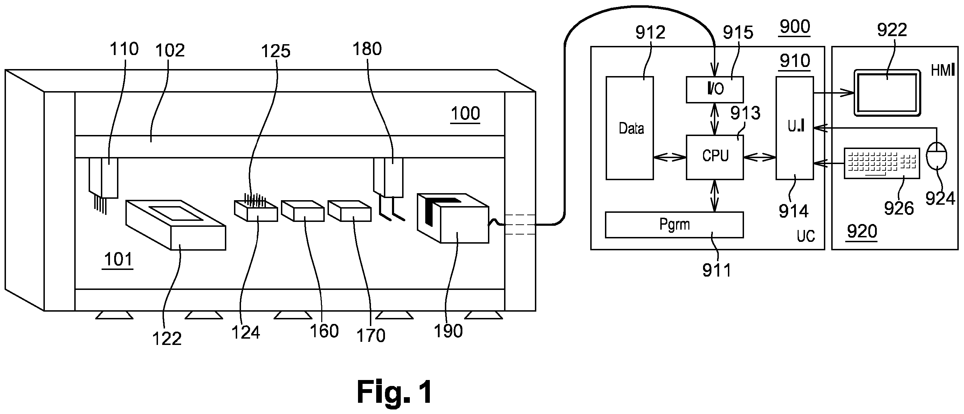

La figure 1 présente un schéma dispositif 100 d'extraction du ou des acides nucléiques du ou des acides nucléiques, de leur purification par séparation et/ou de dosage du ou des acides nucléiques des cibles dans un échantillon contenu dans un organe de réception (non représenté). L'organe de réception sert à réceptionner une quantité d'échantillon à analyser.

Après avoir préparé une quantité d'échantillon, cette dernière est placée dans un organe de réception (non représenté). L'organe de réception contenant une quantité de matrice à analyser. L'ensemble est placé dans une enceinte 101 comprenant des parois latérales, une enceinte délimitée par une partie de parois latérale, une ouverture permettant l'accès à l'enceinte 101 et un volet 102 configuré pour obturer au moins en partie l'ouverture de l'enceinte 101. Le volet 102 est monté pivotant et/ou coulissant par rapport à une paroi latérale de l'enceinte 101. Afin de permettre une visibilité du procédé de détermination et/ou de dosage qui sera exposé ci-après, le volet 102 peut être transparent et/ou translucide. Par ailleurs, étant donné que certains cibles peuvent se situer dans des matrices diverses et variées, tels que du sang, de l'urine et/ou de la fibre musculaire par exemple, le volet 102 doit permettre une séparation de l'enceinte 101 avec le milieu dans lequel le dispositif 100 de détection et/ou de dosage est placé.

L'enceinte 101 est configurée pour permettre la manipulation de l'échantillon et de l'organe de réception (non représenté) à l'intérieur du dispositif 100 de détection et/ou de dosage. Cette manipulation est réalisée par au moins un organe de transfert 110 pouvant être un pipeteur 110 muni de consommables tels que des cônes et/ou d'aiguilles (non représentés). Le pipeteur 110 est configuré pour transférer au moins une partie de la matrice dans la chambre de préparation sans solliciter la présence et/ou l'aide d'une personne. En d'autres termes, le transfert se fait de façon automatique par un automate pipeteur 110. Ainsi, grâce à cette disposition, aucun élément étranger ne peut fausser les résultats obtenus ultérieurement. Le transfert de la quantité d'échantillon effectué par l'organe de transfert 110 se termine dans un premier temps sur un séparateur. Selon un mode de réalisation, l'automate pipeteur 110 peut comprendre un canal ou plusieurs canaux. Ainsi grâce à cette disposition, plusieurs transferts peuvent être effectués en même temps.

Le séparateur est configuré pour séparer les cibles à détecter et/ou à doser dans l'échantillon. Selon un mode de réalisation, le séparateur peut se présenter sous la forme d'une centrifugeuse 122 et/ou une plaque aimantée 124. Cette dernière, c'est-à-dire la plaque aimantée 124, peut comprendre des aimants 125 disposés perpendiculairement à la plaque aimantée 124. De ce fait, un champ magnétique est généré.

Selon les échantillons, qui doivent être manipulées, l'opération de séparation peut être effectuée par centrifugation de sorte à avoir un gradient de séparation entre les corps dit lourds ou relativement lourds qui se trouveront au fond de l'organe de réception (non représenté) et les corps plus légers ou relativement léger par rapport aux corps lourds, qui se trouveront au dessus des corps lourds. Cette centrifugation a lieu lorsque l'organe homogénéisateur met en rotation l'organe de réception (non représenté) par l'intermédiaire d'un plateau tournant autour d'un axe central actionné par un moteur rotatif.

Selon les échantillons qui doivent être manipulés, après les opérations éventuelles de concentration, l'échantillon est placé dans un mix qualifié d'extraction pour en extraire les acides nucléiques.

Selon les matrices qui doivent être manipulées, le mix d'extraction peut contenir diverses substances destinées à provoquer l'éclatement des cellules pour libérer les acides nucléiques.

Les acides nucléiques extraits constituent partie ou totalité des acides nucléiques contenus dans l'échantillon selon la nature du mix d'extraction.

Dans un temps ultérieur il faut extraire de l'ensemble des acides nucléiques la portion spécifique de l'acide nucléique de la cible

En l'espèce, l'opération de séparation visant à isoler l'acide nucléique de la cible peut être effectuée en ajoutant des billes aimantées dans l'organe de réception (non représenté). Les microbilles (non représentées) sont recouvertes de silice pour permettre la fixation de l'ADN extrait sur leurs surfaces extérieures.

Pendant cette phase de purification, l'organe de réception (non représenté) est en position libre hors de la plaque aimantée 124, le temps de mélanger par pipetage et/ou agitation le contenu des puits.

Puis l'organe de réception (non représenté) est amené par le dispositif d'entraînement 180 sur la plaque aimantée 124 de sorte que les microbilles (non représentées) soient attirées sur les bords des puits de l'organe de réception (non représenté) ; par l'organe de transfert 110, on vide le contenu des puits en prenant soin de ne pas toucher les microbilles (non représentées) qui restent sur les parois; par l'organe de transfert 110 on ajoute ensuite un tampon d'élution.

L'organe de réception (non représenté) est alors dégagé de la plaque aimantée 124 par le dispositif d'entraînement 180 pour être placée dans une zone de la chambre de préparation non empreinte de champ magnétique des aimants 125 de la plaque aimantée 124.

Par pipetages successifs, on remet les microbilles (non représentées) en solution dans la solution tampon d'élution.

On remet l'organe de réception (non représenté) sur la plaque aimantée 124 grâce au dispositif d'entraînement 180 ; les microbilles (non représentées) sont plaquées contre les parois des puits de l'organe de réception (non représenté) ; l'ADN est libre dans le tampon d'élution.

Tout ou partie de l'acide nucléique est transférée par l'organe de transfert 110 dans un nouvel organe de réception (non représenté) placé sur un agitateur par le dispositif d'entrainement 180 ; l'organe de transfert 110 ajoute dans chaque puits un mix d'amplification

Le nouvel organe de réception (non représenté) est déplacé par le dispositif d'entraînement 180 à l'emplacement réservé de la scelleuse 170.

Le nouvel organe de réception (non représenté) est recouvert par un film étanche et qui étanchéifie les puits du nouvel organe de réception (non représenté)

Le nouvel organe de réception (non représenté) est placé par le dispositif d'entraînement 180 sur l'emplacement prévu du thermocycleur temps réel 190 ; il est introduit par un dispositif interne du thermocycleur temps réel 190 dans la chambre du thermocycleur temps réel 190.

Le nouvel organe de réception (non représenté) et son contenu sont ensuite soumis aux cycles thermiques du thermocycleur temps réel 190.

Selon la nature de l'échantillon et sa matrice d'origine, le nombre et la durée des cycles thermiques varient entre 10 et 50, le plus souvent entre 30 et 42.

Au cours de ces cycles thermiques les fractions d'ADN sont amplifiées ; au cours de l'amplification, les fluorophores sont excités par la lumière d'excitation ; en réponse, les fluorophores émettent une fluorescence captée par un fluorimètre présent dans le thermocycleur temps réel 190.

Les données sont ensuite collectées par un terminal. Le terminal est typiquement un ordinateur 900 connecté au thermocycleur temps réel 190 par l'intermédiaire d'un câble, d'un réseau intranet, d'un réseau internet et/ou d'un réseau sans fil tels que Wi-Fi™, Bluetooth™, ZigBee®. La figure 1 présente un exemple de connexion entre un thermocycleur temps réel 190 et un terminal. Un tel terminal comporte au moins une unité centrale 910. L'unité centrale 910 comprend au moins un module de programme 911, un moyen de stockage 912 qui peut être matérialisé par un disque dur par exemple, une moyen de connexion 915 permettant la communication d'un terminal avec le thermocycleur temps réel 190, ce moyen de connexion est géré par un module de programme 911 stocké dans la mémoire de ce dernier.

Le terminal 900 possède un moyen d'interaction Homme/Machine 920 - en anglais « Human Machine Interface ». Les moyens d'interaction 920 peuvent comprendre par exemple un écran 922, une souris 924 et/ou un clavier 926.

L'écran 922 permet d'afficher les données transmises par le thermocycleur temps réel 190. Ces données peuvent apparaître à l'écran ou dans une fenêtre n'occupant qu'une partie de l'écran sous forme d'un graphique généré. La génération du graphique est intégrée dans le programme d'interaction que l'utilisateur sélectionne à l'aide d'un navigateur d'application. Après avoir détaillé les différents éléments composant l'invention, nous allons maintenant expliquer comment la détection des cibles est réalisée.

### Présentation des étapes d'extraction du ou des acides nucléiques, de leur purification par séparation et/ou de dosage

Dans une première étape, le module reçoit un ensemble de valeurs détectées par le thermocycleur temps réel 190. Ces valeurs comprennent des valeurs d'intensités de fluorescence par exemple, et des valeurs de cycles et de sorte à former des coordonnées. Ces coordonnées sont uniques car il n'y a pas de redondance en termes de valeurs de cycles. Autrement dit, une valeur de cycle ne peut avoir qu'une et une seule valeur de fluorescence pour un fluorophore donné ; on peut incorporer plusieurs fluorophores dont l'un (ou plus) correspond(ent) à une (ou plusieurs) cible(s), et un autre correspond à un témoin interne. Chaque fluorophore émet une longueur d'ondes propre.

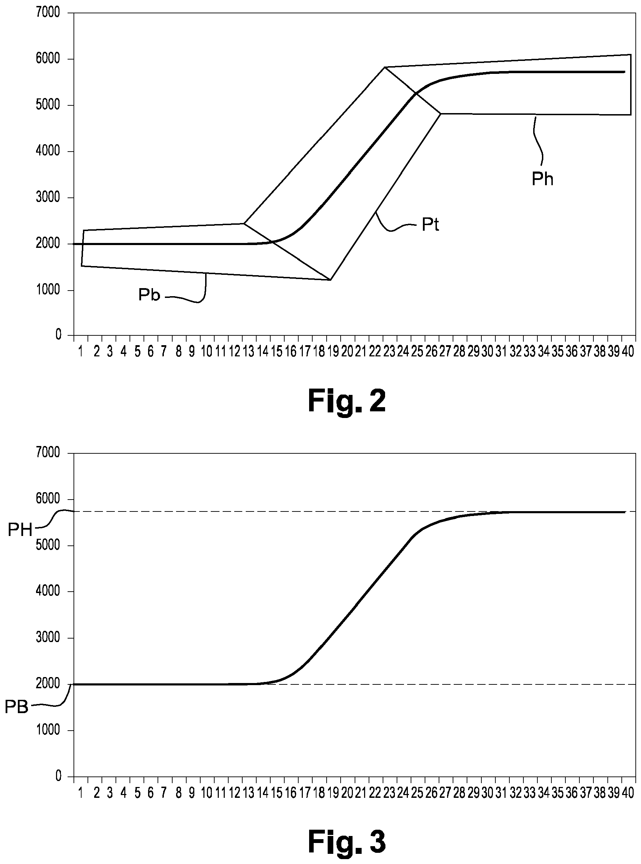

L'ensemble de coordonnées uniques est placé dans un repère orthonormé de sorte à former une courbe de détection. La coordonnée unique comprend, comme écrit précédemment, une intensité de signal de fluorescence. Cette dernière est normée, c'est-à-dire que l'intensité de signal de fluorescence est rapportée à une valeur de référence qui peut être déterminée en amont du procédé. L'intensité de signal de fluorescence normée est placée en fonction d'une valeur unique de cycle d'amplification dans le repère orthonormé de sorte à former la courbe de détection comme représentée sur la figure 2. La courbe de détection dans le repère orthonormé comprend en ordonnée l'intensité de signal de fluorescence normée et en abscisse la valeur de cycle d'amplification.

Afin d'éviter tout écart dû a une éventuelle erreur de mesure, la courbe de détection est lissée. Le lissage de la courbe de détection se produit en effectuant une moyenne de fluorescence d'un cycle d'amplification entre un cycle d'amplification le précédant et un cycle d'amplification le suivant. Ainsi toute erreur de mesure et/ou écart de mesure peut être ainsi lissé(e).

En outre, la courbe de détection se caractérise par le fait (fig. 3) qu'elle comprend une partie basse Pb, une partie haute Ph supérieure, en terme de valeurs d'intensité de signal de fluorescence normées, à la partie basse Pb et une partie transitoire Pt supérieure, en terme de valeurs d'intensité de signal de fluorescence normées, à la partie basse Pb et aussi inférieure, en terme de valeurs d'intensité de signal de fluorescence normées, à la partie haute Ph.

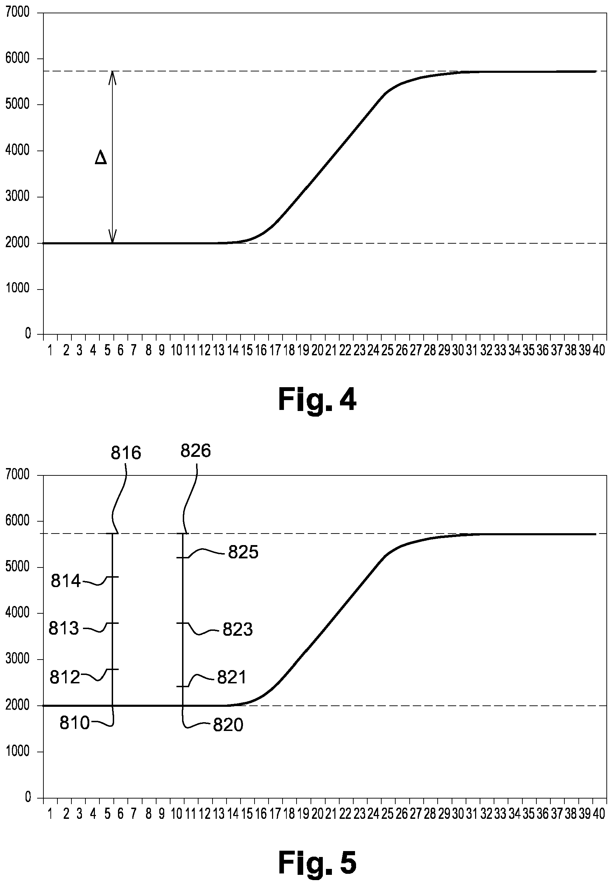

Lorsque la courbe de détection est établie, le module constitue un palier bas PB de fluorescence à partir de la partie basse Pb de la courbe de détection, un palier haut PH à partir de la partie haute Ph de la courbe de détection et calcule la différence Δ d'intensité de fluorescence entre les deux paliers, c'est-à-dire entre le palier bas PB et le palier haut PH (fig. 4).

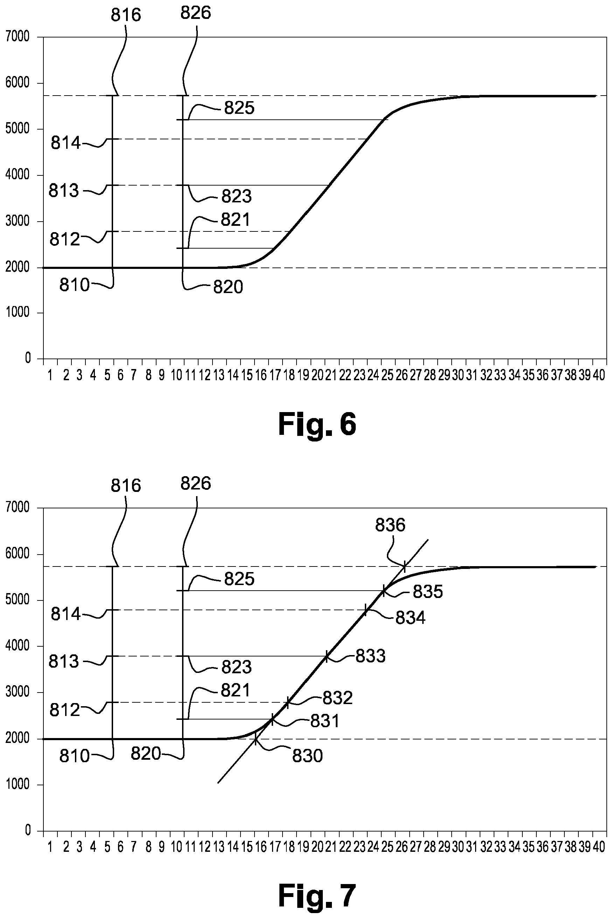

Une fois cette étape terminée, le module fractionne la différence de fluorescence, calculée à l'étape précédente, en portions proportionnelles. Ces portions sont proportionnelles entre elles. Par exemple, si la différence Δ est fractionnée en quatre parties, ces parties peuvent être égales entre elles tels que (810 ; 812) = (812 ; 813) = (813 ; 814) = (814 ; 816) = ¼ Δ ou deux portions pourraient être égales à deux fois les autres tels que (820; 821) = (825 ; 826) = 1/8 Δ et (821 ; 823) = (823 ; 825) = 3/4 Δ. Dans l'exemple représenté sur la figure 6, il sera considéré qu'une portion est égale à deux fois les autres (1/2 Δ = 2*1/4 Δ). Dépendamment de la longueur de la portion, la portion comprend une ordonnée initiale et une ordonnée finale comprises entre le palier bas PB et le palier haut PH de la courbe de détection de sorte à permettre une correspondance entre l'ordonnée initiale et/ou finale d'au moins une portion proportionnelle et l'intensité de signal de fluorescence de la partie transitoire Pt la plus proche de l'ordonnée initiale et/ou finale de la portion proportionnelle.

Lorsque les coordonnées de la partie transitoire Pt sont sélectionnées, le module établit une droite à partir ces coordonnées uniques et prolonge la droite établie de sorte à déterminer un point d'intersection 830 entre la droite prolongée et le palier bas PB.

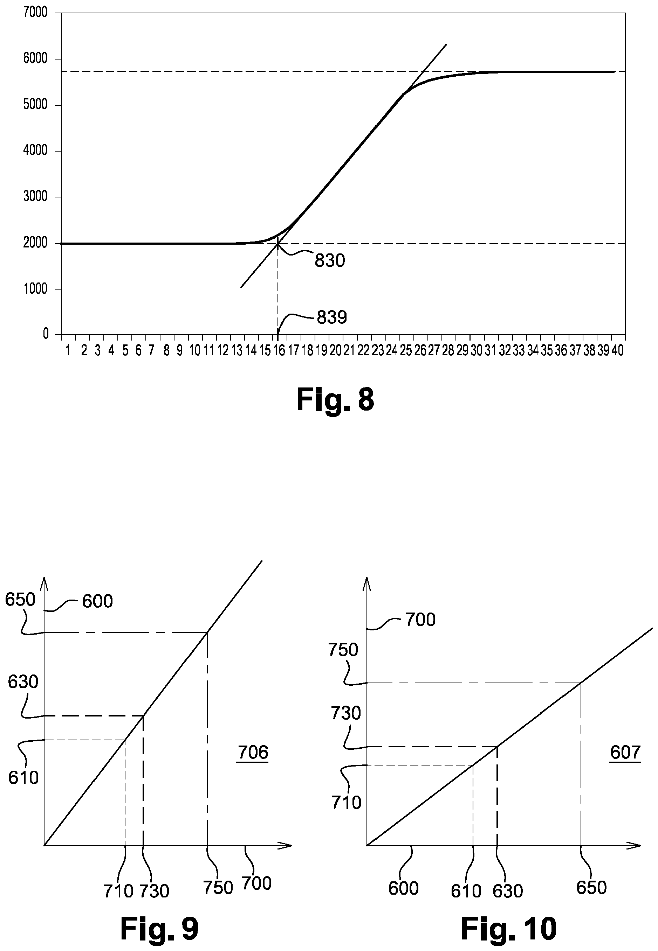

Le module fournit la valeur d'abscisse 839 (fig. 8) correspondant à ce point d'intersection. Cette valeur d'abscisse est positionnée sur une courbe d'étalonnage linéaire 607 et/ou 706 comme représentée sur les figures 9 et 10.

En effet, afin de permettre un dosage des de cibles, différentes solutions témoins, dont les concentrations sont connues, sont dosées comme décrit dans les étapes précédentes. Le module peut ainsi fournir la valeur d'abscisse du point d'intersection en fonction de leurs concentrations ou vice-versa dans une courbe d'étalonnage et par la même occasion fournir une concentration précise des cibles dans la solution à doser.

Ce procédé permet une détection rapide est fiable. En effet, grâce a des étapes de calculs relativement simples, ce procédé permet la détection et/ou le dosage du ou des acides nucléiques d'organismes vivants, pathogènes ou non dans une matrice tout en optimisant les ressources de calcul de l'unité centrale et le nombre de données générées.

Bien que l'invention ait été décrite en liaison avec des exemples particuliers de réalisation, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Dispositif (100) industriel destiné à traiter par automate les échantillons contenant de l'acide nucléique en suivant un processus d'extraction, de purification par séparation, d'amplification pour la détection et/ou le dosage d'acide nucléique de cible(s) dans au moins un échantillon d'au moins une matrice, ledit au moins un échantillon étant contenu dans un organe de réception destiné à être placé dans une enceinte (101) ; ladite enceinte (101) comprend des parois latérales, un plafond, un plancher, une ouverture ; ladite enceinte (101) est configurée pour automatiser la manipulation de l'échantillon à l'intérieur de l'enceinte (101) du dispositif (100) d'extraction, de purification par séparation, d'amplification pour la détection et/ou de dosage du ou des acides nucléiques; ledit dispositif (100) d'extraction, de purification par séparation, d'amplification pour la détection et/ou de dosage comprend :
- au moins un organe de transfert (180) ; l'organe de transfert (180) est configuré pour transférer au moins une partie de l'échantillon dans l'enceinte (101) ;
- au moins un dispositif d'entraînement; le dispositif d'entraînement est configuré pour permettre le déplacement de l'organe de transfert (180) et/ou de l'organe de réception dans l'enceinte (101) ;
- au moins un séparateur ; le séparateur est configuré pour séparer les cibles à détecter et/ou à doser dans l'échantillon ; le séparateur comprend une plaque aimantée comprenant des aimants perpendiculaires à la plaque aimantée ; les aimants sont configurés pour fournir un champ magnétique permettant de séparer, notamment par attraction, les brins d'acides nucléiques de la ou des cibles présentes dans l'échantillon et fixés sur au moins une bille ferromagnétique.
- au moins un homogénéisateur; l'homogénéisateur est configuré pour homogénéiser les cibles à détecter et/ou à doser dans un liquide d'homogénéisation ;
- au moins une scelleuse (170) ; la scelleuse (170) est configurée pour sceller un organe de réception ; et
- au moins un thermocycleur temps réel (190) ; le thermocycleur temps réel (190) est configuré pour permettre une réaction en chaîne par polymérase ;
**caractérisé en ce que**

2. Dispositif 100 selon la revendication 1, dans lequel le thermocycleur temps réel (190) comprend :
- un ensemble de dispositifs de chauffage ; l'ensemble de dispositifs de chauffage permet de faire varier la température de l'organe de réception entre plusieurs températures de cycles ;
- un ensemble de blocs mobiles de chauffage ; l'ensemble de blocs mobiles de chauffage comprend une surface de couplage configurée pour permettre le chauffage de l'organe de réception ;
- un dispositif de mesure ; le dispositif de mesure comprend une source de lumière et un détecteur configuré pour permettre la détection de la lumière émise par les substances chimiques appelées fluorophores et qui ont pour propriété d'émettre une fluorescence de longueur d'onde précise lorsqu'ils reçoivent une excitation par une source de lumière dite d'excitation et dont la longueur d'onde est spécifique pour chaque fluorophore.

3. Dispositif (100) selon la revendication 1, dans lequel l'organe de transfert (180) comprend un automate pipeteur; ledit automate pipeteur comprend au moins un canal ; ledit canal comprend une ouverture, une cavité et un orifice d'admission configuré pour permettre l'admission d'une matière à l'intérieur de la cavité.

4. Dispositif (100) selon la revendication 3, dans lequel le canal est configuré pour permettre l'aspiration d'une matière et/ou le refoulement de la matière comprise à l'intérieur de la cavité.

5. Dispositif (100) selon l'une quelconque des revendications précédentes, lequel comprend en outre un dispositif de transfert (110).

6. Dispositif (100) selon l'une quelconque des revendications précédentes, dans lequel l'organe de réception est configuré pour transmettre une quantité de chaleur entre l'intérieur de l'organe de réception et l'extérieure de l'organe de réception.

7. Dispositif (100) selon l'une quelconque des revendications précédentes, dans lequel l'homogénéisateur comprend:
- un plateau destiné à recevoir au moins un organe de réception servant à réceptionner une quantité de matrice;
- un moteur rotatif configuré pour mettre le plateau en rotation autour d'un axe central.

8. Dispositif (100) selon la revendication précédente, dans lequel le moteur rotatif est configuré pour permettre une rotation comprise entre 0 g et 9000 g

9. Dispositif (100) selon l'une quelconque des revendications précédentes, dans lequel l'organe de réception est configuré pour transmettre une quantité de lumière.

## Patentansprüche

1. Industrielle Vorrichtung (100), die dazu bestimmt ist, mittels Automat die nukleinsäurehaltigen Proben unter Befolgung eines Prozesses der Extraktion, der Aufreinigung mittels Abscheidung, der Amplifikation für den Nachweis und/oder die Dosierung von Nukleinsäure von Target(s) in mindestens einer Probe von mindestens einer Matrix zu verarbeiten, wobei die mindestens eine Probe in einem Aufnahmeorgan enthalten ist, das dazu bestimmt ist, in einem Gehäuse (101) platziert zu werden; wobei das Gehäuse (101) Seitenwände, eine Decke, einen Boden, eine Öffnung umfasst; wobei das Gehäuse (101) dafür konfiguriert ist, die Handhabung der Probe im Inneren des Gehäuses (101) der Vorrichtung (100) zur Extraktion, zur Aufreinigung mittels Abscheidung, zur Amplifikation für den Nachweis und/oder zur Dosierung der Nukleinsäure(n) zu automatisieren; wobei die Vorrichtung (100) zur Extraktion, zur Aufreinigung mittels Abscheidung, zur Amplifikation für den Nachweis und/oder zur Dosierung umfasst:
- mindestens ein Transferorgan (180); wobei das Transferorgan (180) dafür konfiguriert ist, mindestens einen Teil der Probe im Gehäuse (101) zu transferieren;
- mindestens eine Antriebsvorrichtung; wobei die Antriebsvorrichtung dafür konfiguriert ist, das Bewegen des Transferorgans (180) und/oder des Aufnahmeorgans im Gehäuse (101) zu ermöglichen;
- mindestens einen Abscheider; wobei der Abscheider dafür konfiguriert ist, die Targets, die in der Probe nachgewiesen und/oder dosiert werden sollen, abzuscheiden; wobei der Abscheider eine magnetisierte Platte umfasst, die Magnete umfasst, welche zur magnetisierten Platte senkrecht sind; wobei die Magnete dafür konfiguriert sind, ein Magnetfeld zu liefern, das es ermöglicht, insbesondere durch Anziehung die Nukleinsäurestränge des oder der Targets, die in der Probe vorhanden und an mindestens ein ferromagnetisches Bead fixiert sind, abzuscheiden;
- mindestens einen Homogenisator; wobei der Homogenisator dafür konfiguriert ist, die Targets, die nachgewiesen und/oder dosiert werden sollen, in einer Homogenisierungsflüssigkeit zu homogenisieren;
- mindestens einen Versiegler (170); wobei der Versiegler (170) dafür konfiguriert ist, ein Aufnahmeorgan zu versiegeln; und
- mindestens einen Echtzeit-Thermocycler (190); wobei der Echtzeit-Thermocycler (190) dafür konfiguriert ist, eine Polymerasekettenreaktion zu ermöglichen;
**dadurch gekennzeichnet, dass** das Transferorgan einen Pipettierautomaten umfasst, und die Vorrichtung (100) eine Dekontaminationsstation umfasst, die dafür konfiguriert ist zu ermöglichen, die Nadeln des Pipettierautomaten in ihrem Gehäuse (101) zu dekontaminieren.

2. Vorrichtung (100) nach Anspruch 1, wobei der Echtzeit-Thermocycler (190) umfasst:
- einen Satz Heizvorrichtungen; wobei der Satz Heizvorrichtungen es ermöglicht, die Temperatur des Aufnahmeorgans zwischen mehreren Zyklustemperaturen zu variieren;
- einen Satz beweglicher Heizblöcken; wobei der Satz beweglicher Heizblöcke eine Kopplungsfläche umfasst, die dafür konfiguriert ist, das Heizen des Aufnahmeorgans zu ermöglichen;
- eine Messvorrichtung; wobei die Messvorrichtung von eine Lichtquelle und einen Detektor umfasst, der dafür konfiguriert ist, den Nachweis des Lichts zu ermöglichen, das von den chemischen Substanzen emittiert wird, die als Fluorophore bezeichnet werden und die es zur Eigenschaft haben, eine Fluoreszenz einer präzisen Wellenlänge zu emittieren, wenn sie eine Erregung von einer sogenannten Erregungslichtquelle empfangen, und deren Wellenlänge für jedes Fluorophore spezifisch ist.

3. Vorrichtung (100) nach Anspruch 1, wobei das Transferorgan (180) einen Pipettierautomaten umfasst; der Pipettierautomat mindestens einen Kanal umfasst; der Kanal eine Öffnung, einen Hohlraum und ein Einlassloch umfasst, das dafür konfiguriert ist, den Einlass eines Stoffs in das Innere des Hohlraums zu ermöglichen.

4. Vorrichtung (100) nach Anspruch 3, wobei der Kanal dafür konfiguriert ist, das Ansaugen eines Stoffs und/oder das Ausstoßen des Stoffs, der im Inneren des Hohlraums umfasst ist, zu ermöglichen.

5. Vorrichtung (100) nach einem der vorstehenden Ansprüche, die weiter eine Transfervorrichtung (110) umfasst.

6. Vorrichtung (100) nach einem der vorstehenden Ansprüche, wobei das Aufnahmeorgan dafür konfiguriert ist, eine Wärmemenge zwischen dem Inneren des Aufnahmeorgans und der Außenseite des Aufnahmeorgans zu übertragen.

7. Vorrichtung (100) nach einem der vorstehenden Ansprüche, wobei der Homogenisator umfasst:
- einen Teller, der dazu bestimmt ist, mindestens ein Aufnahmeorgan aufzunehmen, das dazu dient, eine Matrixmenge in Empfang zu nehmen;
- einen Drehmotor, der dafür konfiguriert ist, den Teller um eine Mittelachse herum in Drehung zu versetzen.

8. Vorrichtung (100) nach dem vorstehenden Anspruch, wobei der Drehmotor dafür konfiguriert ist, eine Drehung im Bereich zwischen 0 g und 9000 g zu ermöglichen.

9. Vorrichtung (100) nach einem der vorstehenden Ansprüche, wobei das Aufnahmeorgan dafür konfiguriert ist, eine Lichtmenge zu übertragen.

## Claims

1. An industrial device (100) intended to treat by automaton samples containing nucleic acid by following a process of extraction, of purification by separation, of amplification for detection and/or assay of nucleic acid of target(s) in at least one sample of at least one matrix, said at least one sample being contained in a receiving member intended to be placed in an enclosure (101); said enclosure (101) comprises sidewalls, a ceiling, a floor, an opening; said enclosure (101) is configured to automate handling of the sample inside the enclosure (101) of the device (100) of extraction, of purification by separation, of amplification for detection and/or of assay of the nucleic acid(s); said device (100) of extraction, of purification by separation, of amplification for detection and/or of assay comprises:
- at least one transfer member (180); the transfer member (180) is configured to transfer at least one portion of the sample in the enclosure (101);
- at least one drive device; the drive device is configured to enable the displacement of the transfer member (180) and/or of the receiving member in the enclosure (101);
- at least one separator; the separator is configured to separate the targets to be detected and/or to be assayed in the sample; the separator comprises a magnetic plate comprising magnets perpendicular to the magnetic plate; the magnets are configured to provide a magnetic field intended to separate, in particular by attraction, the nucleic acid strands of the target(s) present in the sample and fixed on at least one ferromagnetic ball.
- at least one homogenizer; the homogenizer is configured to homogenize the targets to be detected and/or to be assayed in a homogenization liquid;
- at least one sealer (170); the sealer (170) is configured to seal a receiving member; and
- at least one real-time thermal cycler (190); the real-time thermal cycler (190) is configured to enable a polymerase chain reaction;
**characterized in that**
the transfer member comprises a pipette controller and the device (100) comprises a decontamination station configured to allow the needles of the pipette controller to be decontaminated within its enclosure (101).

2. The device (100) according to claim 1, wherein the real-time thermal cycler (190) comprises:
- a set of heating devices; the set of heating devices allows varying the temperature of the receiving member between several cycle temperatures;
- a set of movable heating blocks; the set of movable heating blocks comprises a coupling surface configured to enable heating of the receiving member;
- a measuring device; the measuring device comprises a light source and a detector configured to enable detection of the light emitted by the chemical substances called fluorophores and which have the property of emitting a fluorescence with an accurate wavelength when they receive an excitation by a called excitation light source whose wavelength is specific for each fluorophore.

3. The device (100) according to claim 1, wherein the transfer member (180) comprises a pipette controller; said pipette controller comprises at least one channel; said channel comprises an opening, a cavity and an intake orifice configured to enable the intake of a material into the cavity.

4. The device (100) according to claim 3, wherein the channel is configured to enable the suction of a material and/or the discharge of the material contained inside the cavity.

5. The device (100) according to any one of the preceding claims, which further comprises a transfer device (110).

6. The device (100) according to any one of the preceding claims, wherein the receiving member is configured to transmit an amount of heat between the inside of the receiving member and the outside of the receiving member.

7. The device (100) according to any one of the preceding claims, wherein the homogenizer comprises:
- a tray intended to receive at least one receiving member for the reception of an amount of matrix;
- a rotary motor configured to rotate the tray about a central axis.

8. The device (100) according to the preceding claim, wherein the rotary motor is configured to enable a rotation between 0 g and 9000 g

9. The device (100) according to any one of the preceding claims, wherein the receiving member is configured to transmit an amount of light.
